# EUROPEAN PATENT APPLICATION

(11) **EP 2 612 664 A1**
(43) Date of publication of application: **10.07.2013**
(21) Application number: 10856710.8
(22) Date of filing: 02.09.2010
(51) Int. Cl.: A61K 31/192, A61K 9/08, A61K 36/53, A61K 36/73, A61K 47/18, A61K 47/22, A61P 27/14, G02C 7/00, G02C 13/00

(54) **STABILIZED POLYPHENOL SOLUTION AND METHOD FOR STABILIZING POLYPHENOL SOLUTION**

(71) Applicant: Menicon Co., Ltd., Nagoya-shi Aichi 460-0006 (JP)
(72) Inventor: HISHIKAWA, Yuri, Nagoya-shi Aichi 460-0006 (JP); OGAWA, Susumu, Kasugai-shi Aichi 487-0032 (JP)
(74) Representative: Gill, Stephen Charles
(86) International application number: PCT/JP2010/065053
(87) International publication number: WO 2012/029160

(57) **Abstract**

There are provided a stabilized polyphenol solution which is applicable as various contact lens solutions and has high lens compatibility and safety, and further also a method for stabilizing such a polyphenol solution. At least one inorganic salt-free buffer is contained in a concentration of 0.001-5% by weight in an aqueous solution containing at least one polyphenol, at least one polyol is further contained therein in a concentration of 0.01-5% by weight to prepare a solution so as to contain no inorganic salt, and the pH of the solution is adjusted to 7.3 or less.

## Description

### TECHNICAL FIELD

The present invention relates to a stabilized polyphenol solution and a method for stabilizing a polyphenol solution, and particularly to a technique for stabilizing a polyphenol in a state of an aqueous solution.

### BACKGROUND ART

A polyphenol has hitherto been expected to be applied to various uses such as cosmetics, pharmaceutical products, quasi-pharmaceutical products, bath salts, foods and drinks, and has been put to practical use, because of its astringent action and skin conditioning-protective action, and further its antioxidant action, eyesight improving effect, vasodilatory effect and the like. Further, it is also proposed to use a soft contact lens solution in which a polyphenol is contained as an allergen-deactivating component, for measures against allergic eye diseases due to allergens such as pollen and house dust in contact lens wearers (PCT International Publication W02007/125731).

By the way, such a polyphenol has a plurality of phenolic hydroxyl groups in its molecule, so that most of them are relatively unstable in a form of an aqueous solution and easily oxidized with oxygen dissolved in water or oxygen in the air to cause a problem of not only a reduction in various useful actions expected for the polyphenol, but also discoloration associated with oxidation.

Then, in JP-A-2008-179547 (Patent Document 1), there has been proposed a solution in which a polyphenol is dissolved in a fatty acid triglyceride in a high concentration and a third component other than the polyphenol is not substantially contained, as a polyphenol preparation excellent in storage stability. Further, in JP-A-6-239716 (Patent Document 2), it is revealed that discoloration of a polyphenol can be prevented by composing a polyphenol aqueous solution so as to contain a compound having two or more alcoholic hydroxyl groups or so as to further contain an organic reducing agent together with such a compound.

However, in these conventional polyphenol stabilizing techniques, an oily solvent of the fatty acid triglyceride is used, or the compound having two or more alcoholic hydroxyl groups is contained in a high concentration. Accordingly, from viewpoints of lens compatibility, safety and the like, it has been difficult to use the solution as a contact lens solution for treating or storing a contact lens.

Further, when the amount of the above-mentioned alcoholic hydroxyl group-containing compound added to the polyphenol aqueous solution is decreased, there has also been inherent a problem that it becomes difficult to sufficiently exhibit the polyphenol stabilizing effect due to such an alcoholic hydroxyl group-containing compound.

### PRIOR ART DOCUMENTS

### PATENT DOCUMENTS

Patent Document 1: JP-A-2008-179547
Patent Document 2: JP-A-6-239716

### SUMMARY OF INVENTION

### TECHNICAL PROBLEM

The present invention has been made herein in the light of such situations, and objects thereof is to provide a stabilized polyphenol solution which is applicable as various contact lens solutions and has high lens compatibility and safety, and further also to provide a method for stabilizing such a polyphenol solution.

### SOLUTION TO PROBLEM

Then, in order to achieve the objects described above, or to solve problems understood from the description of the entire specification and the drawings, the present invention can be suitably carried out in various aspects described below, and the respective aspects described below can also be employed in any combination. It is to be understood that the aspects and technical features of the present invention are not limited to those described below and should be recognized on the basis of the description of the entire specification or the concept of the invention disclosed therein.

(1) A stabilized polyphenol solution comprising an aqueous solution which contains at least one polyphenol, characterized in that the solution contains no inorganic salt, and contains at least one inorganic salt-free buffer in a concentration of 0.001-5% by weight and at least one polyol in a concentration of 0.01-5% by weight; and the pH of the solution is adjusted to 7.3 or less.
(2) The stabilized polyphenol solution according to the above aspect (1), wherein the buffer is a Good's buffer.
(3) The stabilized polyphenol solution according to the above aspect (1) or (2), wherein the polyol is selected from glycerol and propylene glycol.
(4) The stabilized polyphenol solution according to any one of the above aspects (1) to (3), wherein the polyphenol is contained in a concentration of 0.00001-10% by weight.
(5) The stabilized polyphenol solution according to any one of the above aspects (1) to (4), wherein the polyphenol is selected from perilla extract, butterbur extract and rose extract.
(6) The stabilized polyphenol solution according to any one of the above aspects (1) to (4), wherein the polyphenol is rosmarinic acid or luteolin.
(7) The stabilized polyphenol solution according to any one of the above aspects (1) to (6), wherein the solution is used as a contact lens solution which is brought into contact with a contact lens.
(8) The stabilized polyphenol solution according to any one of the above aspects (1) to (6), wherein the solution is used as an additive solution to a contact lens solution which is brought into contact with a contact lens.
(9) The stabilized polyphenol solution according to the above aspect (7) or (8), wherein the contact lens solution is a multipurpose solution, an enzyme-containing cleaning solution, a storage solution or a packaging solution.
(10) The stabilized polyphenol solution according to any one of the above aspects (7) to (9), wherein the contact lens is a soft contact lens.
(11) The stabilized polyphenol solution according to the above aspect (10), wherein the soft contact lens is a nonionic contact lens.
(12) A method for stabilizing a polyphenol solution comprising an aqueous solution which contains at least one polyphenol, characterized in that the solution is composed so as to contain no inorganic salt, and contain at least one inorganic salt-free buffer in a concentration of 0.001-5% by weight and at least one polyol in a concentration of 0.01-5% by weight; and the pH of the solution is adjusted to 7.3 or less.

### ADVANTAGEOUS EFFECTS OF INVENTION

As described above, in the stabilized polyphenol solution and the method for stabilizing a polyphenol solution according to the present invention, the inorganic salt-free buffer and the polyol are both contained in small proportions in the aqueous solution of the polyphenol, and further, the pH of the solution is adjusted to about neutrality or the acidic side. Thus, the solution is prepared so as to contain substantially no inorganic salt, thereby being able to effectively stabilize the polyphenol, which can advantageously inhibit or prevent the occurrence of a trouble such as denaturation, even in an aqueous medium.

Moreover, as well as the content of such an inorganic salt-free buffer, the content of the polyol is as low as 5% by weight or less. Accordingly, the stabilized polyphenol solution according to the present invention has high safety to the eye and also has excellent compatibility to the contact lens, thereby being able to be suitably used as the contact lens solution.

### DESCRIPTION OF EMBODIMENTS

By the way, in the stabilized polyphenol solution according to the present invention, at least one predetermined polyphenol is contained in an aqueous medium, and the polyphenol used therein is a plant constituent having a plurality of phenolic hydroxyl groups in its molecule. All of the conventionally known polyphenols can be employed, and examples thereof include flavones, flavonols, isoflavones, flavans, flavanols, flavanones, flavanonols, chalcones, anthocyanidins, phenylcarboxylic acid-based polyphenols and the like. Further, examples thereof include tea extracts (including sweet tea), and plant extract components other than the tea extracts, such as persimmons (fruits, leaves and trees), bamboos, low striped bamboos, butterburs, perillas, roses, gardenia fruits, ginkgoes, aloes and rosemaries. Among them, particularly, rosmarinic acid or luteolin, or perilla extract containing them as representative components, or butterbur extract, rose extract or the like is preferably used. The polyphenols as described above can be used either alone or as a combination of two or more thereof.

Then, by adding the polyphenol as described above into an appropriate aqueous medium in the same manner as conventional one so as to be contained therein, the polyphenol-containing aqueous solution used in the present invention can be prepared. As the aqueous medium used in that case, there can be utilized any one, as well as water itself such as tap water, purified water or distilled water, as long as it is a substantially inorganic salt-free solution mainly composed of water, has high safety to living organisms and is sufficiently ophthalmologically allowable.

Here, the content of such a polyphenol in the aqueous solution is appropriately selected depending on its intended use. However, it is generally within the range of 0.00001-10%, and preferably within the range of 0.01-5%, on the weight basis. When the content is excessively decreased, there is a possibility that the action and effect of the polyphenol added cannot be sufficiently exhibited. On the other hand, when the content is excessively increased, there is a possibility that a problem of lens coloration or the like is encountered, in the case where used as a contact lens solution.

Then, in the present invention, both of an inorganic salt-free buffer and a polyol are contained in the polyphenol-containing aqueous solution obtained as described above at predetermined rates, respectively, thereby being able to realize the polyphenol solution having excellent stability.

The inorganic salt-free buffer used herein is a buffer in which no inorganic salt is contained as one of its constituents, and at least one selected from various conventionally known buffers can be appropriately used. In particular, a Good's buffer is suitably used in the present invention. The Good's buffers are selected from various known ones such as TAPS, Bis-Tris, HEPES, MES, ADA, PIPES, ACES, BES and TES, and used either alone or as a combination of two or more thereof.

Such an inorganic salt-free buffer is contained in a concentration of 0.001-5%, preferably in a concentration of about 0.01-2%, on the weight basis, in the polyphenol-containing aqueous solution. When the concentration of such a buffer is excessively decreased, it becomes difficult to exhibit the effect of sufficiently stabilizing the solution. On the other hand, when the concentration thereof is excessively increased, a problem of toxicity of the buffer itself is encountered to cause a problem also in use thereof as the contact lens solution.

Further, the polyol is a compound having two or more alcoholic hydroxyl groups, and at least one is appropriately selected from various conventionally known ones. Among them, glycerol or propylene glycol can be advantageously used.

Then, such a polyol is contained in a concentration of 0.01-5%, preferably in a concentration of about 0.1-2%, on the weight basis, in the polyphenol-containing aqueous solution. When the concentration of the polyol is excessively decreased, it becomes difficult to expect the effect of sufficiently stabilizing the solution, which is exhibited by combination use with the above-mentioned inorganic salt-free buffer. On the other hand, when the concentration thereof is excessively increased, a problem of increased osmotic pressure or the like is encountered, in addition to the problem of toxicity.

As described above, the polyphenol solution according to the present invention is prepared such that both of the inorganic salt-free buffer and the polyol are contained in the polyphenol-containing aqueous solution at predetermined rates, respectively. Accordingly, no inorganic salt is contained in such a solution, thereby being able to advantageously contribute to the stabilization of the polyphenol solution. Further, the pH of such a solution is adjusted to 7.3 or less, thereby being able to further enhance the stabilization of the polyphenol solution.

In order to adjust the pH of the polyphenol-containing aqueous solution containing the inorganic salt-free buffer and the polyol to 7.3 or less, various known pH adjusting agents can be used. It is necessary to avoid the use of such pH adjusting agents causes an inorganic salt to be present in the solution. In general, such pH adjustment can be easily realized by combining a plurality of inorganic salt-free buffers. When such a pH of the solution is excessively decreased, a possibility of adversely affecting an object such as the contact lens becomes liable to occur. Accordingly, the lower limit of such a pH is defined to be about 4.

Further, the polyphenol solution according to the present invention thus prepared may further contain various conventionally known addition components, as needed, in addition to the contained components as described above. For example, in the contact lens solution use, one or two or more of addition components such as a thickening agent or a thickener, a chelating agent, a cooling agent, an enzyme, a cleaning agent, a surfactant and an osmoregulating agent may be appropriately selected and contained at usual contents. However, it goes without saying that it should be avoided to use a component which causes the inorganic salt to be present in the polyphenol solution by addition thereof as such an addition component (additive).

Then, the polyphenol solution stabilized according to the present invention can be used for various uses, so that the effective functions of the polyphenol solution acts on or is imparted to various objects. In the present invention, however, the polyphenol solution is suitably used as the contact lens solution which is brought into contact with the contact lens. Then, when used as the contact lens solution, the polyphenol solution can be applied to the contact lens in various conventionally known forms, and is generally used as a wearing solution for wearing the contact lens and further as an eyewash, eye drops or the like, as well as used as a multi-purpose solution, an enzyme-containing cleaning solution, a storage solution or a packaging solution. The function or action of the polyphenol can be advantageously imparted to the contact lens by immersing the contact lens in these solutions. The multi-purpose solution as used herein means a solution in which at least two or more of cleaning, rinsing, disinfection and storage can be performed by a single solution.

Further, as the contact lens to which such a polyphenol solution according to the present invention is suitably applied, there is advantageously used a soft contact lens. The soft contact lens to which such a polyphenol solution is applied includes various known contact lenses. For example, nonionic soft contact lenses include a soft contact lens composed of a vinyl acetate-based or dimethyl acrylamide-based polymer, or a polymer of a silicon-containing methacrylic monomer, which is called a silicone hydrogel. Further, ionic soft contact lenses include a soft contact lens composed of a HEMA-based, N-VP-based or MAA-based polymer in which ionic groups such as carboxyl groups are introduced. In particular, among these soft contact lenses, the polyphenol solution according to the present invention is advantageously applied to the nonionic soft contact lenses.

As described above, by using the polyphenol solution according to the present invention as the soft contact lens solution, such a polyphenol is advantageously incorporated into such a lens, when it comes into contact with the lens. The polyphenol incorporated is continuously released little by little on the eye over a long period of time, and thereby, it is unnecessary to newly apply (supply) the polyphenol, even when an allergen such as pollen is newly deposited on the lens. Accordingly, allergic symptoms such as hay fever can be advantageously decreased or relieved by a decreased application times.

Further, when the polyphenol solution according to the present invention is brought into contact with the contact lens such as the soft contact lens, such a contact lens is immersed in the polyphenol solution, or the polyphenol solution is deposited on the lens. The contact time of the contact lens with the polyphenol solution is generally from about 30 minutes to 24 hours. When the contact time becomes short, it becomes difficult to sufficiently expect the action and effect due to the polyphenol. On the other hand, when the contact time becomes excessively long, it becomes difficult to sufficiently expect the effect associated therewith.

As described above, it becomes possible to obtain the soft contact lens containing the sustained-releasable polyphenol by bringing the contact lens, particularly the soft contact lens, into contact with the polyphenol solution according to the present invention, and introducing the polyphenol component into such a soft contact lens.

The polyphenol solution according to the present invention can be suitably used as the contact lens solution as it is. In addition, it can also be advantageously used as an additive solution to such a contact lens solution. That is to say, it is also possible to allow the function or action of the polyphenol to be exhibited together, in a form in which such a polyphenol solution is added to the multi-purpose solution, the enzyme-containing cleaning solution, the storage solution, the packaging solution, the wearing solution or the like such that the polyphenol is contained in each solution, using each solution for a use similar to the conventional one.

### EXAMPLES

The present invention will be clarified more specifically below, showing some examples of the present invention. However, it goes without saying that the present invention is not limited by the description of such examples. Moreover, it should be understood that in addition to the following examples, various changes, modifications, improvements and the like can be made in the present invention, based on the knowledge of those skilled in the art, without departing from the spirit of the present invention.

The pH of each test solution in the following examples was measured by using a pH meter (manufactured by Horiba, Ltd., F-24). For appearance observation, each test solution was put in a colorless, transparent glass tube having a diameter of about 2 cm, and the presence or absence of coloration thereof was evaluated against a background of white paper. Further, the absorbance of each test solution was determined by measuring the absorbance thereof in a region of 280-330 nm using an ultraviolet-visible spectrophotometer (manufactured by Shimadzu Corporation, UV-3150).

Then, a high-pressure steam sterilization treatment in the following examples was performed by sterilizing each test solution by a process of steaming under pressure at a temperature of 121°C for 20 minutes using a high-pressure steam sterilizer (manufactured by Hirayama Manufacturing Corp., HVE-50). The pH, the appearance and the absorbance of each test solution before and after this high-pressure steam sterilization treatment were evaluated as described above. For a change in pH, when the change in pH after the high-pressure steam sterilization treatment was less than 0.5 as compared with the pH immediately after the preparation of the solution, it was evaluated as "good", and when the change in pH was 0.5 or more, it was evaluated as "poor". Further, for a change in appearance, when the solution after the high-pressure steam sterilization treatment had no change as compared with the appearance immediately after the preparation of the solution and no coloration was observed, it was evaluated as "good", and when coloration was observed, it was evaluated as "poor". Furthermore, in the measurement of the absorbance, the absorbance in a region of 280-330 nm was measured immediately after the preparation of the solution. When the solution after the high-pressure steam sterilization treatment had a peak height exceeding 70% as compared with the peak height at a wavelength of maximum absorption, it was evaluated as "good", and when the solution had a peak height of 70% or less, it was evaluated as "poor". When there was no change in peak height and there was a peak shift, it was also evaluated as "poor".

Further, with respect to evaluation of an accelerated storage treatment for each test solution in the following examples, each test solution was accommodated in a temperature and humidity testing chamber (manufactured by Nagano Science Co., Ltd., LH21-13M) set at a temperature of 45°C and a relative humidity of 50%, and stored under the set conditions for 1 month, thereby performing the accelerated storage treatment. Changes in pH, appearance and peak height at a wavelength of maximum absorption in a region of 280-330 nm before and after the treatment were examined and evaluated in the same manner as in the case of the above-mentioned high-pressure steam sterilization treatment.

In addition, with respect to comprehensive evaluation for each test solution in the following examples, when of a total of 6 evaluation items in the above-mentioned high-pressure steam sterilization treatment and accelerated storage treatment, a total of 5 or more evaluation items were evaluated as "good", it was rated as "good". On the other hand, when the number of evaluation items evaluated as "good" was less than 5, it was rated as "poor".

### -Example 1-

Various test solutions 1A to 1 P using rosmarinic acid as a polyphenol were prepared as described below, and thereafter, the respective test solutions were subjected to the high-pressure steam sterilization treatment and the accelerated storage treatment. Changes of the respective test solutions before and after these treatments (changes in pH, changes in appearance and changes in peak height at a wavelength of maximum absorption in a region of 280-330 nm) were examined. The results thereof are shown in the following Table 1, together with compositions of the respective test solutions.

Test solution 1A: Rosmarinic acid was dissolved in an aqueous solution containing 2% glycerol and 0.05% TAPS to a concentration of 0.01 %, and thereafter, the pH thereof was adjusted to 4.5 using a 0.05% Bis-Tris aqueous solution, thereby obtaining test solution 1A containing no inorganic salt.
Test solution 1 B: Rosmarinic acid was dissolved in the same manner as in the above-mentioned test solution 1A, and thereafter, the pH thereof was adjusted to 5.0 using a 0.05% Bis-Tris aqueous solution, thereby obtaining test solution 1 B containing no inorganic salt.
Test solution 1C: Rosmarinic acid was dissolved in the same manner as in the above-mentioned test solution 1A, and thereafter, the pH thereof was adjusted to 5.5 using a 0.05% Bis-Tris aqueous solution, thereby obtaining test solution 1C containing no inorganic salt.
Test solution 1 D: Rosmarinic acid was dissolved in the same manner as in the above-mentioned test solution 1A, and thereafter, the pH thereof was adjusted to 6.5 using a 0.05% Bis-Tris aqueous solution, thereby obtaining test solution 1 D containing no inorganic salt.
Test solution 1E: Rosmarinic acid was dissolved in an aqueous solution containing 2% glycerol and 1% TAPS to a concentration of 0.01 %, thereby obtaining test solution 1 E containing no inorganic salt.
Test solution 1F: Rosmarinic acid was dissolved in an aqueous solution containing 0.01 % glycerol and 0.05% TAPS to a concentration of 0.01 %, and thereafter, the pH thereof was adjusted to 5.5 using a 0.05% Bis-Tris aqueous solution, thereby obtaining test solution 1 F containing no inorganic salt.
Test solution 1G: Rosmarinic acid was dissolved in an aqueous solution containing 2% glycerol and 0.01 % TAPS to a concentration of 0.01 %, thereby obtaining test solution 1 G containing no inorganic salt.
Test solution 1H: Rosmarinic acid was dissolved in an aqueous solution containing 0.9% NaCl as an inorganic salt and 0.03% EDTA to a concentration of 0.01 %, thereby obtaining test solution 1 H.
Test solution 1I: Rosmarinic acid was dissolved in an aqueous solution containing 0.6% disodium hydrogen phosphate and 0.05% sodium dihydrogen phosphate as buffers, further 0.83% NaCl as an inorganic salt and 0.03% EDTA to a concentration of 0.01 %, thereby obtaining test solution 1I.
Test solution 1J: Rosmarinic acid was dissolved in an aqueous solution containing 2% glycerol and 0.05% TAPS to a concentration of 0.01 %, and thereafter, the pH thereof was adjusted to 7.5 using a 0.05% Bis-Tris aqueous solution, thereby obtaining test solution 1J containing no inorganic salt.
Test solution 1K: Rosmarinic acid was dissolved in a 1% Bis-Tris aqueous solution to a concentration of 0.01%, thereby obtaining test solution 1 K containing no inorganic salt.
Test solution 1 L: Rosmarinic acid was dissolved in a 2% glycerol aqueous solution to a concentration of 0.01 %, thereby obtaining test solution 1 L containing no inorganic salt.
Test solution 1M: Rosmarinic acid was dissolved in a 2% propylene glycol aqueous solution to a concentration of 0.01%, thereby obtaining test solution 1M containing no inorganic salt.
Test solution 1N: Rosmarinic acid was dissolved in a 1% HEPES aqueous solution to a concentration of 0.01%, thereby obtaining test solution 1 N containing no inorganic salt.
Test solution 1O: Rosmarinic acid was dissolved in a 1% TAPS aqueous solution to a concentration of 0.01 %, thereby obtaining test solution 1O containing no inorganic salt.
Test solution 1P: Rosmarinic acid was dissolved in an aqueous solution containing 0.001 % glycerol and 0.05% TAPS to a concentration of 0.01 %, and thereafter, the pH thereof was adjusted to 5.5 using a 0.05% Bis-Tris aqueous solution, thereby obtaining test solution 1 P containing no inorganic salt.

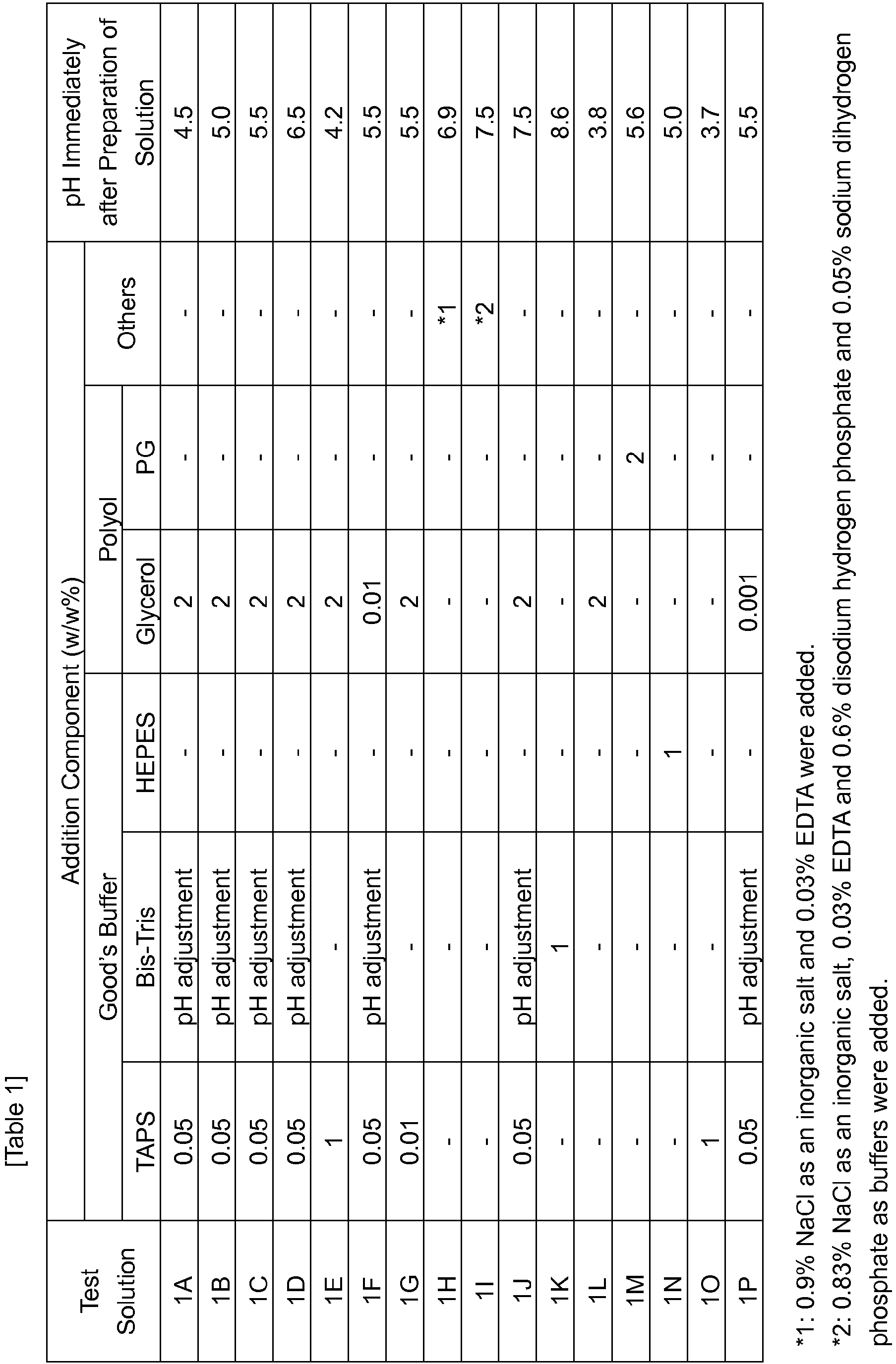

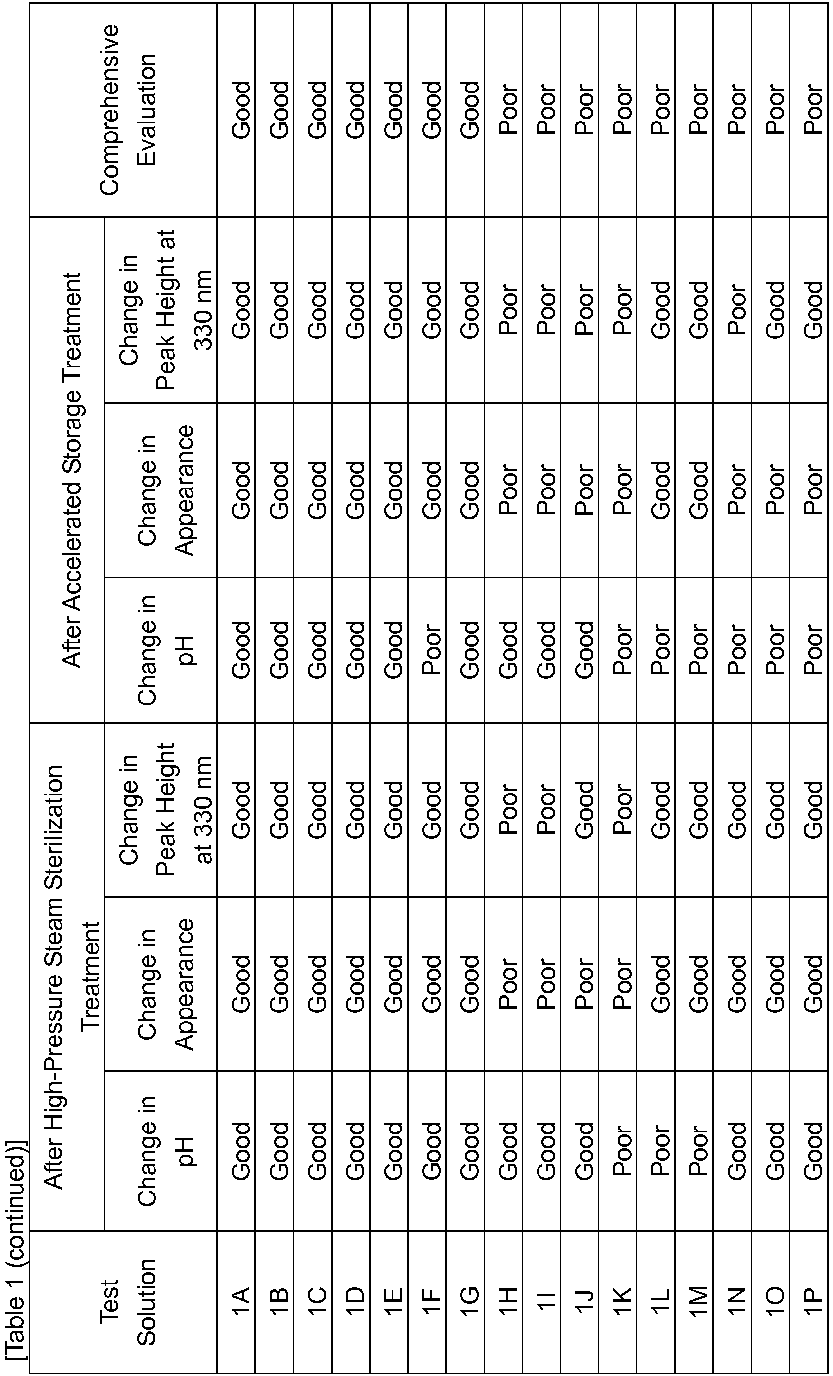

As is apparent from the results of Table 1, it was revealed that test solutions 1A to 1 G according to the present invention were all excellent in their stability even when subjected to the high-pressure steam sterilization treatment or the accelerated storage treatment. In contrast, it was revealed that all the cases where the Good's buffers or the polyols were each separately used even when they were used, the test solutions not containing them at all, further the test solutions having a pH exceeding 7.3 and the test solutions using the inorganic salt or the buffer containing the inorganic salt lacked stability and involved problems of the change in pH, coloration, the change in peak height and the like.

### -Example 2-

Various test solutions 2A to 2H using luteolin as a polyphenol were prepared as described below, and thereafter, the respective test solutions were subjected to the high-pressure steam sterilization treatment and the accelerated storage treatment. Changes of the respective test solutions before and after these treatments (changes in pH, changes in appearance and changes in peak height at a wavelength of maximum absorption in a region of 280-330 nm) were examined. The results thereof are shown in the following Table 2, together with compositions of the respective test solutions.

Test Solution 2A: Luteolin was dissolved in an aqueous solution containing 2% glycerol and 1% TAPS to a concentration of 0.01 %, thereby obtaining test solution 2A containing no inorganic salt.
Test Solution 2B: Luteolin was dissolved in an aqueous solution containing 0.9% NaCl as an inorganic salt and 0.03% EDTA to a concentration of 0.01%, thereby obtaining test solution 2B.
Test Solution 2C: Luteolin was dissolved in an aqueous solution containing 0.6% disodium hydrogen phosphate and 0.05% sodium dihydrogen phosphate as buffers, further 0.83% NaCl as an inorganic salt and 0.03% EDTA to a concentration of 0.01 %, thereby obtaining test solution 2C.
Test Solution 2D: Luteolin was dissolved in a 1% Bis-Tris aqueous solution to a concentration of 0.01 %, thereby obtaining test solution 2D containing no inorganic salt.
Test Solution 2E: Luteolin was dissolved in a 2% glycerol aqueous solution to a concentration of 0.01 %, thereby obtaining test solution 2E containing no inorganic salt.
Test Solution 2F: Luteolin was dissolved in a 2% propylene glycol aqueous solution to a concentration of 0.01%, thereby obtaining test solution 2F containing no inorganic salt.
Test Solution 2G: Luteolin was dissolved in a 1% HEPES aqueous solution to a concentration of 0.01 %, thereby obtaining test solution 2G containing no inorganic salt. Test Solution 2H: Luteolin was dissolved in a 1% TAPS aqueous solution to a concentration of 0.01 %, thereby obtaining test solution 2H containing no inorganic salt.

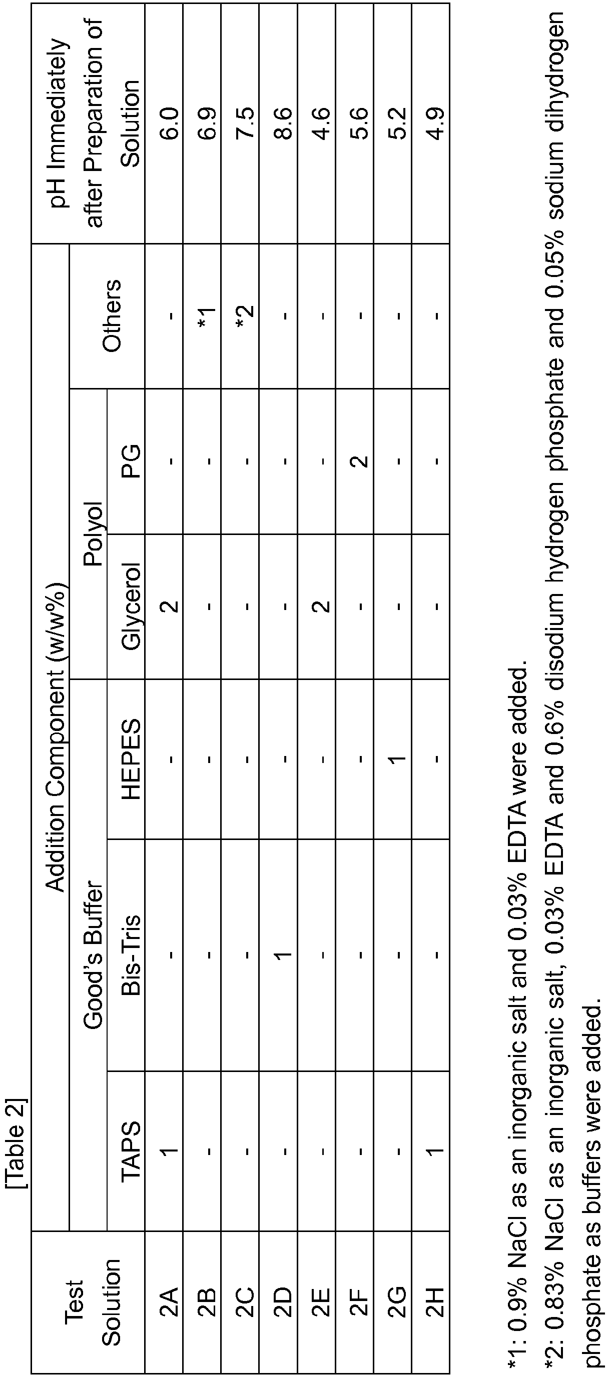

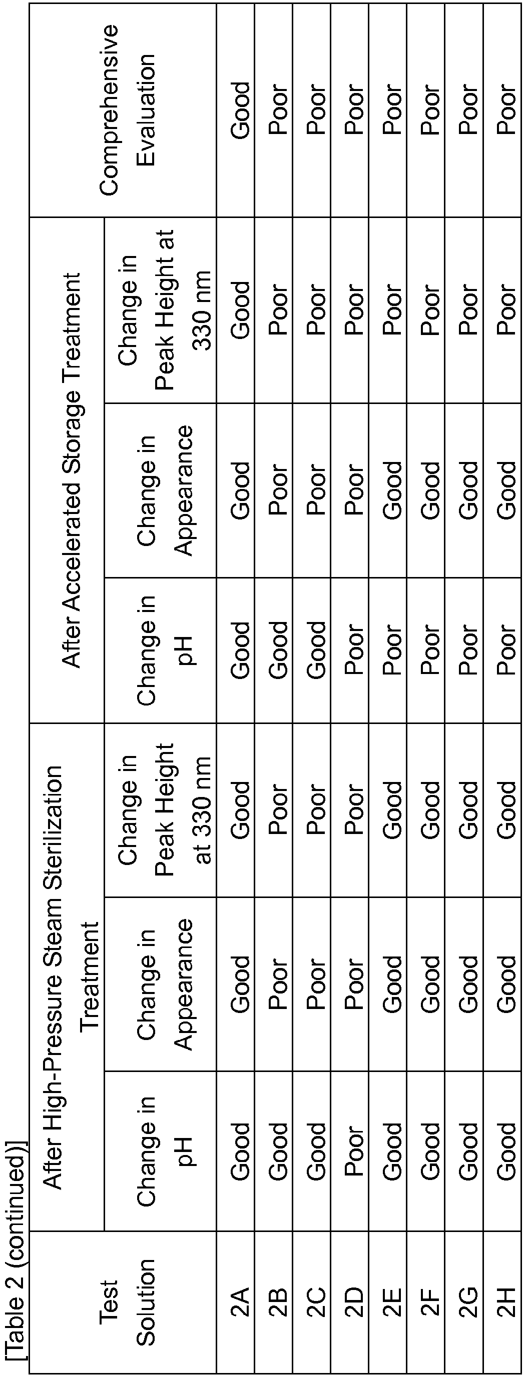

As is apparent from the results of Table 2, it was revealed that test solution 2A according to the present invention was excellent in its stability even when subjected to the high-pressure steam sterilization treatment or the accelerated storage treatment. In contrast, it was revealed that all the cases where the Good's buffers or the polyols were each separately used even when they were used, the test solutions not containing them at all, further the test solutions having a pH exceeding 7.3 and the test solutions using the inorganic salt or the buffer containing the inorganic salt lacked stability and involved problems of the change in pH, coloration, the change in peak height and the like.

### -Example 3-

Various test solutions 3A to 3I using polyphenol-containing perilla extract were prepared as described below, and thereafter, the respective test solutions were subjected to the high-pressure steam sterilization treatment and the accelerated storage treatment. Changes of the respective test solutions before and after these treatments (changes in pH, changes in appearance and changes in peak height at a wavelength of maximum absorption in a region of 280-330 nm) were examined. The results thereof are shown in the following Table 3, together with compositions of the respective test solutions.

Test Solution 3A: Perilla extract was dissolved in an aqueous solution containing 2% glycerol and 1% TAPS to a concentration of 0.01 %, thereby obtaining test solution 3A containing no inorganic salt.
Test Solution 3B: Perilla extract was dissolved in an aqueous solution containing 2% propylene glycol and 1% TAPS to a concentration of 0.01 %, thereby obtaining test solution 3B containing no inorganic salt.
Test Solution 3C: Perilla extract was dissolved in an aqueous solution containing 0.9% NaCl as an inorganic salt and 0.03% EDTA to a concentration of 0.01 %, thereby obtaining test solution 3C.
Test Solution 3D: Perilla extract was dissolved in an aqueous solution containing 0.6% disodium hydrogen phosphate and 0.05% sodium dihydrogen phosphate as buffers, further 0.83% NaCl as an inorganic salt and 0.03% EDTA to a concentration of 0.01 %, thereby obtaining test solution 3D.
Test Solution 3E: Perilla extract was dissolved in a 1% Bis-Tris aqueous solution to a concentration of 0.01%, thereby obtaining test solution 3E containing no inorganic salt.
Test Solution 3F: Perilla extract was dissolved in a 2% glycerol aqueous solution to a concentration of 0.01%, thereby obtaining test solution 3F containing no inorganic salt.
Test Solution 3G: Perilla extract was dissolved in a 2% propylene glycol aqueous solution to a concentration of 0.01%, thereby obtaining test solution 3G containing no inorganic salt.
Test Solution 3H: Perilla extract was dissolved in a 1% HEPES aqueous solution to a concentration of 0.01%, thereby obtaining test solution 3H containing no inorganic salt.
Test Solution 3I: Perilla extract was dissolved in a 1% TAPS aqueous solution to a concentration of 0.01 %, thereby obtaining test solution 3I containing no inorganic salt.

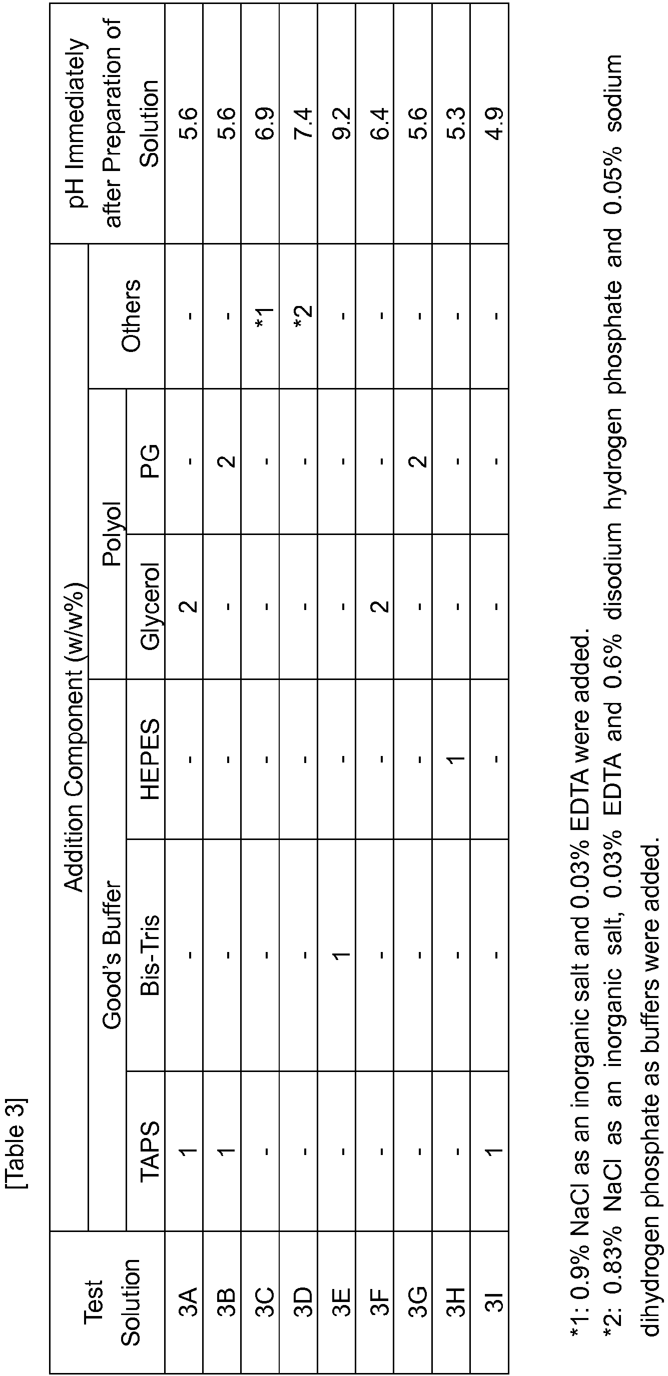

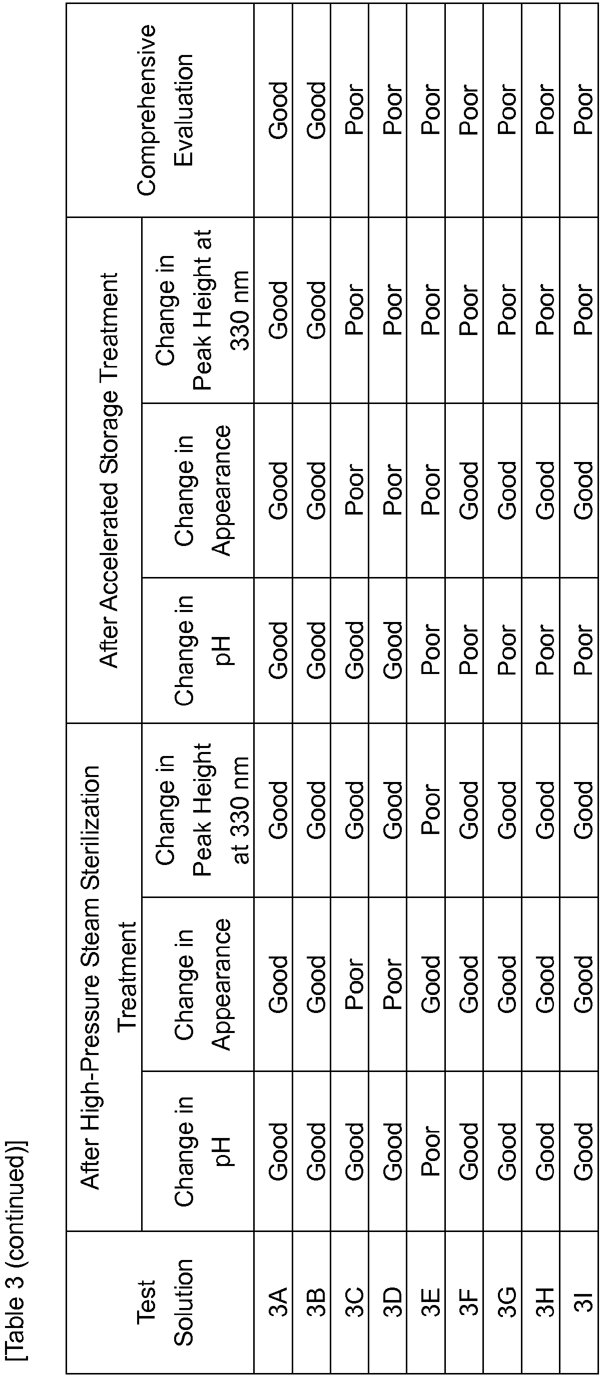

As is apparent from the results of Table 3, it was revealed that test solutions 3A and 3B according to the present invention were both excellent in their stability even when subjected to the high-pressure steam sterilization treatment or the accelerated storage treatment. In contrast, it was revealed that all the cases where the Good's buffers or the polyols were each separately used even when they were used, the test solutions not containing them at all, further the test solutions having a pH exceeding 7.3 and the test solutions using the inorganic salt or the buffer containing the inorganic salt lacked stability and involved problems of the change in pH, coloration, the change in peak height and the like.

### -Example 4-

Various test solutions 4A to 4I using polyphenol-containing rose extract were prepared as described below, and thereafter, the respective test solutions were subjected to the high-pressure steam sterilization treatment and the accelerated storage treatment. Changes of the respective test solutions before and after these treatments (changes in pH, changes in appearance and changes in peak height at a wavelength of maximum absorption in a region of 280-330 nm) were examined. The results thereof are shown in the following Table 4, together with compositions of the respective test solutions.

Test Solution 4A: Rose extract was dissolved in an aqueous solution containing 2% glycerol and 1% TAPS to a concentration of 0.01 %, thereby obtaining test solution 4A containing no inorganic salt.
Test Solution 4B: Rose extract was dissolved in an aqueous solution containing 2% propylene glycol and 1% TAPS to a concentration of 0.01 %, thereby obtaining test solution 4B containing no inorganic salt.
Test Solution 4C: Rose extract was dissolved in an aqueous solution containing 0.9% NaCl as an inorganic salt and 0.03% EDTA to a concentration of 0.01 %, thereby obtaining test solution 4C.
Test Solution 4D: Rose extract was dissolved in an aqueous solution containing 0.6% disodium hydrogen phosphate and 0.05% sodium dihydrogen phosphate as buffers, further 0.83% NaCl as an inorganic salt and 0.03% EDTA to a concentration of 0.01 %, thereby obtaining test solution 4D.
Test Solution 4E: Rose extract was dissolved in a 1% Bis-Tris aqueous solution to a concentration of 0.01%, thereby obtaining test solution 4E containing no inorganic salt.
Test Solution 4F: Rose extract was dissolved in a 2% glycerol aqueous solution to a concentration of 0.01%, thereby obtaining test solution 4F containing no inorganic salt.
Test Solution 4G: Rose extract was dissolved in a 2% propylene glycol aqueous solution to a concentration of 0.01%, thereby obtaining test solution 4G containing no inorganic salt.
Test Solution 4H: Rose extract was dissolved in a 1% HEPES aqueous solution to a concentration of 0.01%, thereby obtaining test solution 4H containing no inorganic salt.
Test Solution 4I: Rose extract was dissolved in a 1 % TAPS aqueous solution to a concentration of 0.01 %, thereby obtaining test solution 4I containing no inorganic salt.

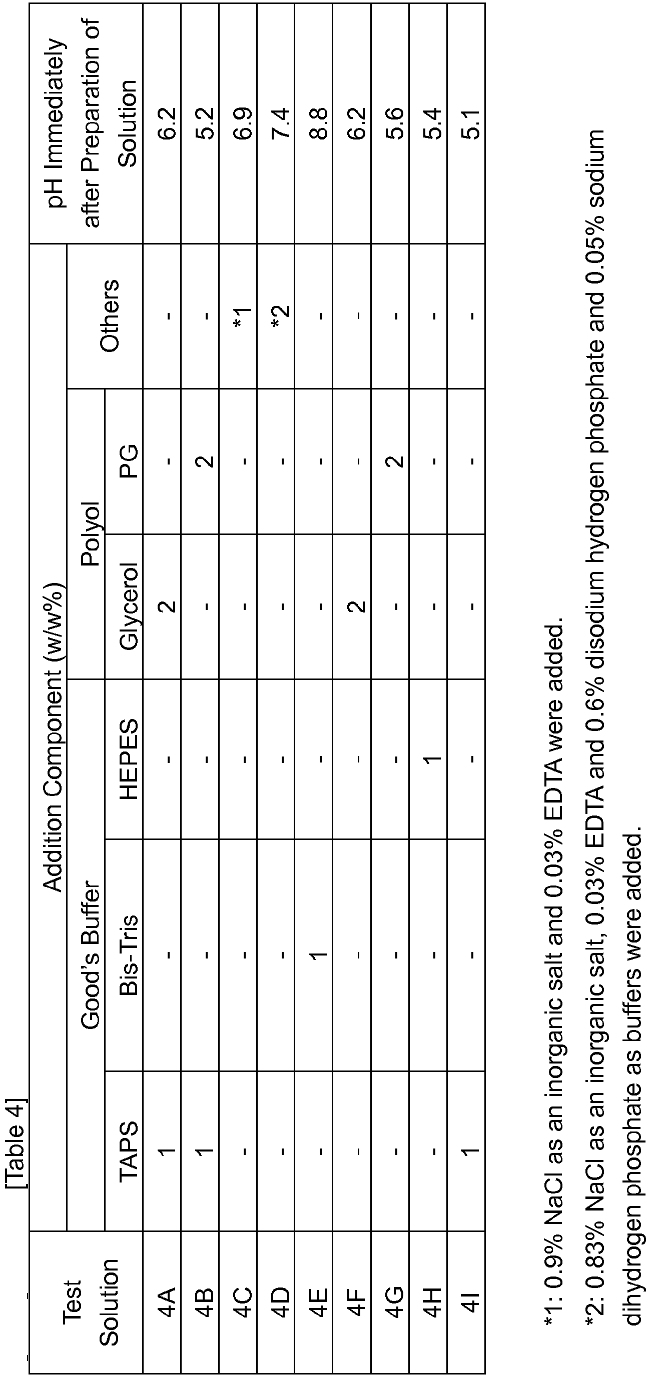

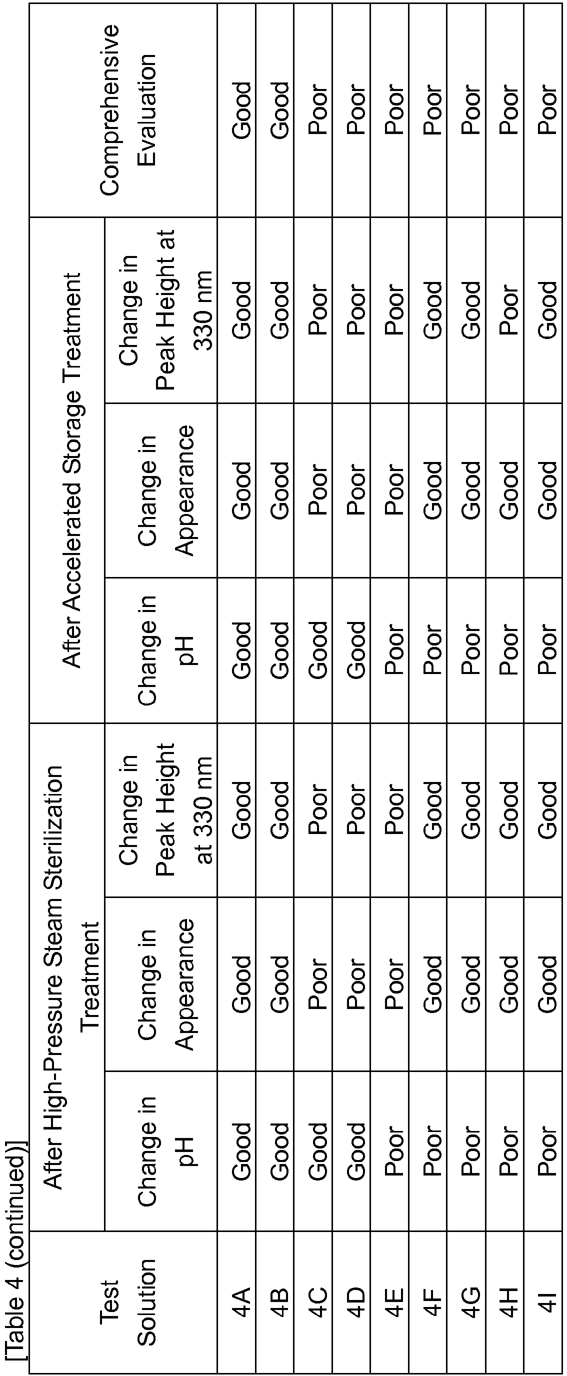

As is apparent from the results of Table 4, it was revealed that test solutions 4A and 4B according to the present invention were both excellent in their stability even when subjected to the high-pressure steam sterilization treatment or the accelerated storage treatment. In contrast, it was revealed that all the cases where the Good's buffers or the polyols were each separately used even when they were used, the test solutions not containing them at all, further the test solutions having a pH exceeding 7.3 and the test solutions using the inorganic salt or the buffer containing the inorganic salt lacked stability and involved problems of the change in pH, coloration, the change in peak height and the like.

### -Example 5-

Various test solutions 5A to 5I using polyphenol-containing butterbur extract were prepared as described below, and thereafter, the respective test solutions were subjected to the high-pressure steam sterilization treatment and the accelerated storage treatment. Changes of the respective test solutions before and after these treatments (changes in pH, changes in appearance and changes in peak height at a wavelength of maximum absorption in a region of 280-330 nm) were examined. The results thereof are shown in the following Table 5, together with compositions of the respective test solutions.

Test Solution 5A: Butterbur extract was dissolved in an aqueous solution containing 2% glycerol and 1% TAPS to a concentration of 0.01%, thereby obtaining test solution 5A containing no inorganic salt.
Test Solution 5B: Butterbur extract was dissolved in an aqueous solution containing 2% propylene glycol and 1% TAPS to a concentration of 0.01%, thereby obtaining test solution 5B containing no inorganic salt.
Test Solution 5C: Butterbur extract was dissolved in an aqueous solution containing 0.9% NaCl as an inorganic salt and 0.03% EDTA to a concentration of 0.01 %, thereby obtaining test solution 5C.
Test Solution 5D: Butterbur extract was dissolved in an aqueous solution containing 0.6% disodium hydrogen phosphate and 0.05% sodium dihydrogen phosphate as buffers, further 0.83% NaCl as an inorganic salt and 0.03% EDTA to a concentration of 0.01 %, thereby obtaining test solution 5D.
Test Solution 5E: Butterbur extract was dissolved in a 1% Bis-Tris aqueous solution to a concentration of 0.01%, thereby obtaining test solution 5E containing no inorganic salt.
Test Solution 5F: Butterbur extract was dissolved in a 2% glycerol aqueous solution to a concentration of 0.01%, thereby obtaining test solution 5F containing no inorganic salt.
Test Solution 5G: Butterbur extract was dissolved in a 2% propylene glycol aqueous solution to a concentration of 0.01%, thereby obtaining test solution 5G containing no inorganic salt.
Test Solution 5H: Butterbur extract was dissolved in a 1% HEPES aqueous solution to a concentration of 0.01%, thereby obtaining test solution 5H containing no inorganic salt.
Test Solution 5I: Butterbur extract was dissolved in a 1% TAPS aqueous solution to a concentration of 0.01%, thereby obtaining test solution 5I containing no inorganic salt.

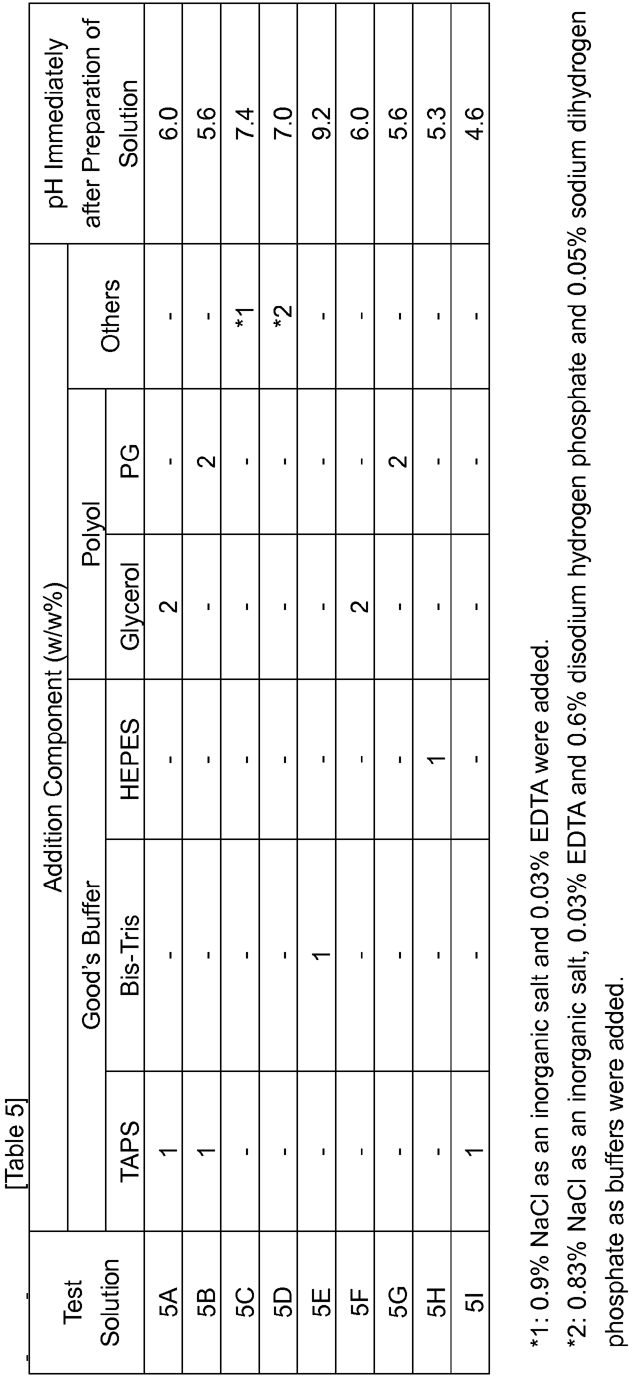

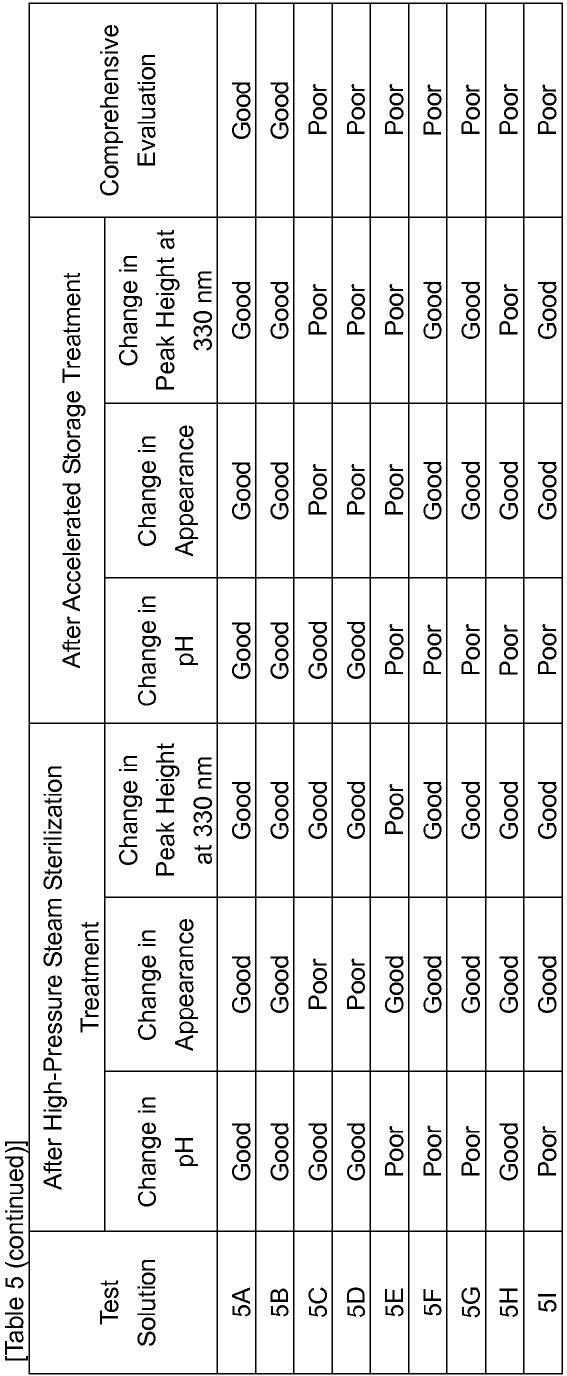

As is apparent from the results of Table 5, it was revealed that test solutions 5A and 5B according to the present invention were both excellent in their stability even when subjected to the high-pressure steam sterilization treatment or the accelerated storage treatment. In contrast, it was revealed that all the cases where the Good's buffers or the polyols were each separately used even when they were used, the test solutions not containing them at all, further the test solutions having a pH exceeding 7.3 and the test solutions using the inorganic salt or the buffer containing the inorganic salt lacked stability and involved problems of the change in pH, coloration, the change in peak height and the like.

### -Example 6-

Using test solution 1A prepared in the above-described Example 1 (0.01 % rosmarinic acid + 2% glycerol + 0.05% TAPS; adjusted to pH 4.5 with the 0.05% Bis-Tris aqueous solution), rosmarinic acid (polyphenol) uptake and release tests were performed to two kinds of commercially available contact lenses. As the commercially available contact lenses, there were used "1-DAY ACUVUE" (commercial product A: manufactured by Johnson & Johnson Vision Care), ionic soft contact lenses, and "PremiO" (commercial product B: manufactured by Menicon Co., Ltd.), nonionic soft contact lenses.

Then, 2mL of the above-mentioned test solution 1A was accommodated in each of two lens vials, and thereafter, each piece of the above-mentioned two kinds of commercially available lenses was further immersed therein. Similarly, two pieces of the respective kinds of lenses were each immersed in 2mL of test solution 1A accommodated in each of lens vials. For the respective kinds of lenses, three lens vials were prepared. Then, the three lens vials for the respective kinds of lenses, in which test solution 1A was accommodated and in which each piece of the two kinds of commercially available lenses was immersed, were left at the room temperature for 2 days.

### (1) Rosmarinic Acid Release Test

First, rosmarinic acid was dissolved in saline, and thereafter, 0.1, 0.25, 0.5, 1.0, 2.50 and 5.0µg/mL rosmarinic acid standard solutions (saline) were prepared by serial dilution. Then, the absorbance thereof in a region of 280-330 nm was measured, and a calibration curve was prepared from the absorbance at the wavelength of maximum absorption and the concentration.

On the other hand, the above-mentioned respective lenses left at the room temperature for 2 days were taken out of the lens vials, and immersed in 10mL of saline after rinsing lightly. After 0.5, 1, 2, 3, 6 and 24 hours, 600µL of the saline in which the lenses were each immersed was collected, and 600µL of fresh saline was added thereto. Then, for the saline collected, the absorbance in a region of 280-330 nm was measured, and the absorbance at the wavelength of maximum absorption was substituted in the calibration curve prepared above to calculate the concentration of rosmarinic acid in each saline collected. The results thereof are shown in the following Table 6.

**[Table 6]**

| Immersion Time [hr] | Release Amount [µg] | | | |
|---|---|---|---|---|
| | Commercial Product A | | Commercial Product B | |
| | Average | Standard Deviation | Average | Standard Deviation |
| 0.5 | 0.5 | 0.10 | 7.6 | 2.0 |
| 1 | 1.5 | 0.17 | 11.0 | 1.3 |
| 2 | 1.8 | 0.21 | 20.6 | 1.5 |
| 3 | 1.9 | 0.24 | 31.1 | 0.5 |
| 6 | 2.0 | 0.29 | 35.4 | 0.6 |
| 24 | 2.2 | 0.33 | 36.5 | 0.7 |

### (2) Rosmarinic Acid Uptake Test

Further, the uptake amount (introduction amount) of rosmarinic acid in the above-mentioned two kinds of commercially available contact lenses was examined as described below.

First, using the above-mentioned test solution 1A, rosmarinic acid was dissolved therein, and thereafter, 25, 50 and 100µg/mL rosmarinic acid standard solutions were prepared by serial dilution. Then, the absorbance thereof in a region of 280-330 nm was measured, and a calibration curve was prepared from the absorbance at the wavelength of maximum absorption and the concentration.

On the other hand, for test solution 1A in the lens vial after the lens was taken out thereof, the absorbance in a region of 280-330 nm was measured, and the absorbance at the wavelength of maximum absorption was substituted in the calibration curve prepared above to determine the residual concentration of rosmarinic acid in test solution 1A in the lens vial. Then, the uptake amount of rosmarinic acid taken up by each of commercial products A and B was calculated from the concentration of rosmarinic acid in test solution 1A before the release test. The results thereof are shown in the following Table 7.

**[Table 7]**

| Uptake Amount [µg] | | | |
|---|---|---|---|
| Commercial Product A | | Commercial Product B | |
| Average | Standard Deviation | Average | Standard Deviation |
| 15 | 1.2 | 50 | 0.0 |

As is apparent from the results of Tables 6 and 7, it is recognized that a predetermined amount of rosmarinic acid in the test solution 1A is taken up by both of commercial products A and B which are soft contact lenses, and that the rosmarinic acid taken up is gradually released from both of commercial products A and B with time. It is understood that pharmacological effects due to rosmarinic acid can be advantageously exhibited thereby over a long period of time.

## Claims

1. A stabilized polyphenol solution comprising an aqueous solution which contains at least one polyphenol, **characterized in that** the solution contains no inorganic salt, and contains at least one inorganic salt-free buffer in a concentration of 0.001-5% by weight and at least one polyol in a concentration of 0.01-5% by weight; and the pH of the solution is adjusted to 7.3 or less.

2. The stabilized polyphenol solution according to claim 1, wherein the buffer is a Good's buffer.

3. The stabilized polyphenol solution according to claim 1 or 2, wherein the polyol is selected from glycerol and propylene glycol.

4. The stabilized polyphenol solution according to any one of claims 1 to 3, wherein the polyphenol is contained in a concentration of 0.00001-10% by weight.

5. The stabilized polyphenol solution according to any one of claims 1 to 4, wherein the polyphenol is selected from perilla extract, butterbur extract and rose extract.

6. The stabilized polyphenol solution according to any one of claims 1 to 4, wherein the polyphenol is rosmarinic acid or luteolin.

7. The stabilized polyphenol solution according to any one of claims 1 to 6, wherein the solution is used as a contact lens solution which is brought into contact with a contact lens.

8. The stabilized polyphenol solution according to any one of claims 1 to 6, wherein the solution is used as an additive solution to a contact lens solution which is brought into contact with a contact lens.

9. The stabilized polyphenol solution according to claim 7 or 8, wherein the contact lens solution is a multipurpose solution, an enzyme-containing cleaning solution, a storage solution or a packaging solution.

10. The stabilized polyphenol solution according to any one of claims 7 to 9, wherein the contact lens is a soft contact lens.

11. The stabilized polyphenol solution according to claim 10, wherein the soft contact lens is a nonionic contact lens.

12. A method for stabilizing a polyphenol solution comprising an aqueous solution which contains at least one polyphenol, **characterized in that** the solution is composed so as to contain no inorganic salt, and contain at least one inorganic salt-free buffer in a concentration of 0.001-5% by weight and at least one polyol in a concentration of 0.01-5% by weight; and the pH of the solution is adjusted to 7.3 or less.
